# EUROPEAN PATENT APPLICATION

(11) **EP 4 180 015 A1**
(43) Date of publication of application: **17.05.2023**
(21) Application number: 21207967.7
(22) Date of filing: 12.11.2021
(51) Int. Cl.: A61F 13/15, B26D 1/01, B26D 1/45, B26D 5/08, B26D 7/08

(54) **SYSTEM AND METHOD FOR THE SHAPED CUTTING OF SANITARY ARTICLES, PARTICULARLY OF WASHABLE ABSORBENT GARMENTS**

(71) Applicant: Fameccanica.Data S.p.A., 66020 San Giovanni Teatino (CH) (IT)
(72) Inventor: LUPINETTI, Serafino, I-66020 San Giovanni Teatino (Chieti) (IT)
(74) Representative: Marchitelli, Mauro

(57) **Abstract**

System and method for the shaped cutting of sanitary articles having a back layer (12) of washable fabric and the like, wherein the back layer (12) is fed longitudinally along a travel direction to a cutting unit (30) including at least one cutting tool (34) movable transversally to the travel direction so as to cut at least one longitudinal side edge of the back layer (12), in combination with the longitudinal displacement thereof, along a curved profile.

## Description

### Field of the invention

The present invention generally relates to sanitary articles and the like for the absorption and retention of liquids, and more particularly -though not exclusively- to washable and reusable absorbent garments comprising a back layer made of washable fabric.

More specifically the invention is directed to a system and to a method for the shaped cutting of the back layer of such washable absorbent garments, having a pant structure formed by rear and front waist sections and a crotch section intermediate between the rear and front waist sections.

### Prior art

In the production of washable absorbent garments the fabric back layer is fed longitudinally along a travel direction to a cutting station including cutting tools movable transversally to said travel direction so as to cut each longitudinal side edge of the web, in combination with the displacement the web and, along curved profiles defining said crotch sections.

Each shaped cutting tool traditionally consists of a mechanical rotating blade (commonly defined as a roller knife), acting on a smooth contrast roller better defined as a counter knife or anvil.

Dimensions, limits of use and costs of such known cutting tools are essentially influenced by the type of raw material to be cut, by the process speed and, last but not least, by the type of steel used to produce the cutting blade, as well as clearly by the skill of the manufacturer.

The limitations regarding the aforementioned conventional technology essentially concern the lack of flexibility in the format changes, where at least the roller knife must be replaced. Therefore, it is necessary to always make at least two roller knives available in the warehouse for each format (one to be used when necessary and one as a spare). It goes without saying that considering the costs of all the knives and the downtime necessary for the integration of the format change unit into the machine, the known solution is of little advantage, especially in a context where frequent format change would be required.

### Object and summary of the invention

The object of the present invention is to provide a system and method for the shaped cutting of sanitary articles which overcome the above drawbacks of the prior art.

According to the present invention, this object is achieved by cutting system whose main features are set forth in claim 1 and by a cutting method as primarily defined in claims 10.

The invention has been conceived to carry out, on a specific layer for instance made of fabric sheet, nonwoven, polyethylene film, etc., a shaped cutting with a continuous and/or discrete intermittent profile, the shape of which is obtained through the dynamic vectorial correlation between the instantaneous speed of the layer and the position of the sonic punch or the laser beam moved by scanners or fixed optical systems in a dynamic shape transversal direction with respect to that of the advancement of the layer under treatment, through the use of suitably proportioned motors or actuators.

In both cases, the cutting tools must act on an adequate support system, capable of guaranteeing adequate rigidity, inertia, and position of the treated material.

The system and method of the invention are released from any replacement of parts during the format change phase, as the latter can be achieved thanks to the loading on the electronic control unit of the cutting system of a different SW/Motion file specific to the cutting profile to be created. This dramatically reduces downtime and at the same time increases the flexibility of the cutting process.

The appended claims form an integral part of the disclosure provided here in relation to the invention.

### Brief description of the drawings

The invention will now be described in detail with reference to the attached drawings, given purely by way of non-limiting example, wherein:
- Figure 1 is a diagrammatic front elevational view of part of a cutting system according to a first embodiment the invention,
- Figure 2 is an enlarged view of part of Figure 1,
- Figure 3 is a view same as Figure 2 showing a second embodiment of the invention,
- Figure 4 is a perspective enlarged view of an example of one cutting tool employed by the invention,
- Figure 5 is a perspective view of one example of washable absorbent garment for which the invention can be carried out, and
- Figure 6 is a plan view of Figure 5.

It will be appreciated that the various figures may not be represented on the same scale. Some dimensions and dimensional ratios are being enlarged for the sake of better understanding. It will also be appreciated that in some figures certain elements or components may not be shown, also for a better understanding.

### Detailed description

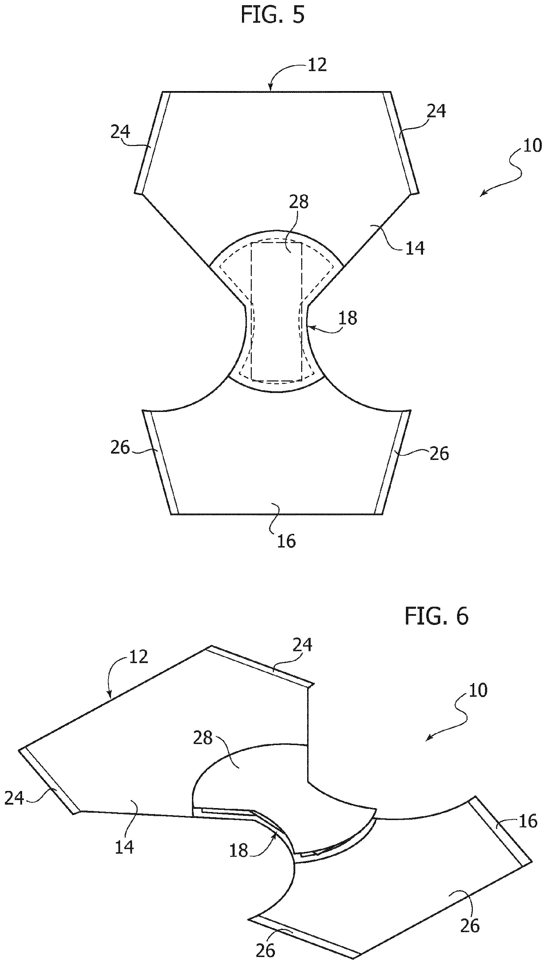

With reference initially to Figures 5 and 6 the numeral reference 10 indicates an example of a washable absorbent garment for which the invention can be carried out to advantage. It is to be pointed out that such a garment is just exemplary since the invention can be equally employed with different garments having any shaped profile similar or comparable to the one disclosed hereinafter.

The washable absorbent garment 10 comprises a back layer 12 of washable fabric forming a pant structure. The back layer 12 has rear and front waist sections 14, 16 and a crotch section 18 intermediate between the rear and front waist sections 14, 16. The rear and front waist sections 14, 16 are wider than the crotch section 18, such that in the extended position of figures 5 and 6 the back layer 12 has substantially an hourglass shape.

The back layer 12 is a fabric formed by woven threads which is washable as ordinary textile garments, e.g. swimsuits, underwear, etc. The back layer 12 may be made of any natural or synthetic material used for producing textile articles, e.g. cotton, Lycra, nylon, polyester, polypropylene etc. and any mixture thereof. In possible embodiments, the back layer 12 may be an elastic fabric made of a mixture of polyamide and elastane or cotton and elastane, e.g. 80% cotton/polyamide and 20% elastane. The back layer 20 may have for instance a weight comprised between 100-200 g/m².

The crotch section 18 of the back layer 12 has two curved side edges 22 shaped to conform to the legs of the user in the configuration in which the washable absorbent garment 10 is worn. The rear and front waist sections 14, 16 of the back layer 12 have respective side edges 24, 26 which are joined to each other such that the washable absorbent garment 10 is closed in a pant-like shape and has two leg openings on opposite sides of the crotch section 18.

The side edges 24, 26 are joined to each other by ultrasonic welding or by glue.

The washable absorbent garment 10 also comprises a washable absorbent core 28 permanently fixed in the crotch section 18 of the back layer 12.

Production of the washable absorbent garment 10 is carried out by unwinding the back layer 12 from a reel and advancing it continuously (or even intermittently) in generally horizontal machine direction. The individual absorbent cores 28 are provided on a parallel line and are permanently fixed onto the back layer in longitudinally spaced positions to form a continuous garment tape.

The continuous tape formed by the back layer 12 carrying the absorbent cores 28 is fed to a transversally movable cutting unit 30 configured for cutting the longitudinal side edges of the back layer 12, as the continuous garment tape advances in the machine direction, along curved profiles shaped to conform to the legs of the user. The cutting tools are movable transversally to the travel direction of the continuous tape so as to cut each longitudinal side edge of the tape, in combination with the longitudinal displacement of the tape, along such curved profiles to provide the crotch sections 18 of the washable absorbent garments 10.

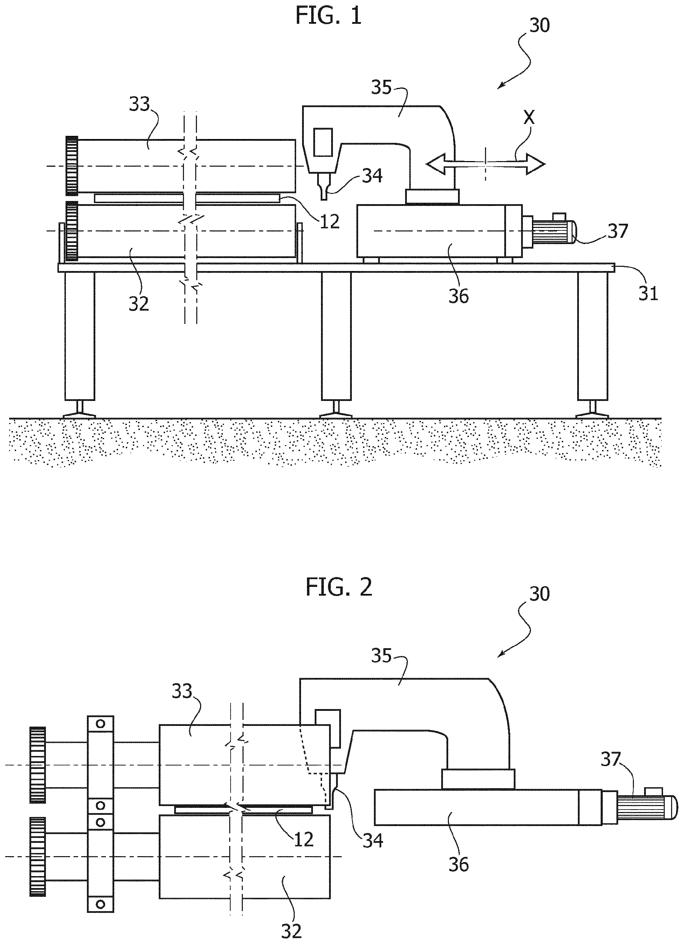

Referring now to Figure 1, the cutting unit 30 comprises a support frame 31 bearing a pair of juxtaposed cutting tools, operating jointly or even alternatively at the opposite sides of the back layer 12, only one of which is depicted for the sake of simplicity in Figures 1-3.

It is to pointed out that the cutting unit 30 could be equipped with just one cutting tool in case the continuous layer of a sanitary article different from garment 10 is to be cut only along one of its longitudinal edges.

The cutting unit 30 is displaceable transversally, in the direction of arrows X, with respect to travel direction of the continuous tape formed by the back layer 12 carrying the absorbent cores 28, which is advanced longitudinally by a motor-driven conveyor including for instance pairs of transverse superimposed rollers 32, 33. Along the path aside the cutting unit 30 the support frame 31 can bear a backing plate or a flat anvil roller upon which cutting of the back layer 12 is performed, so as to provide the necessary rigidity and proper positioning of the material being cut.

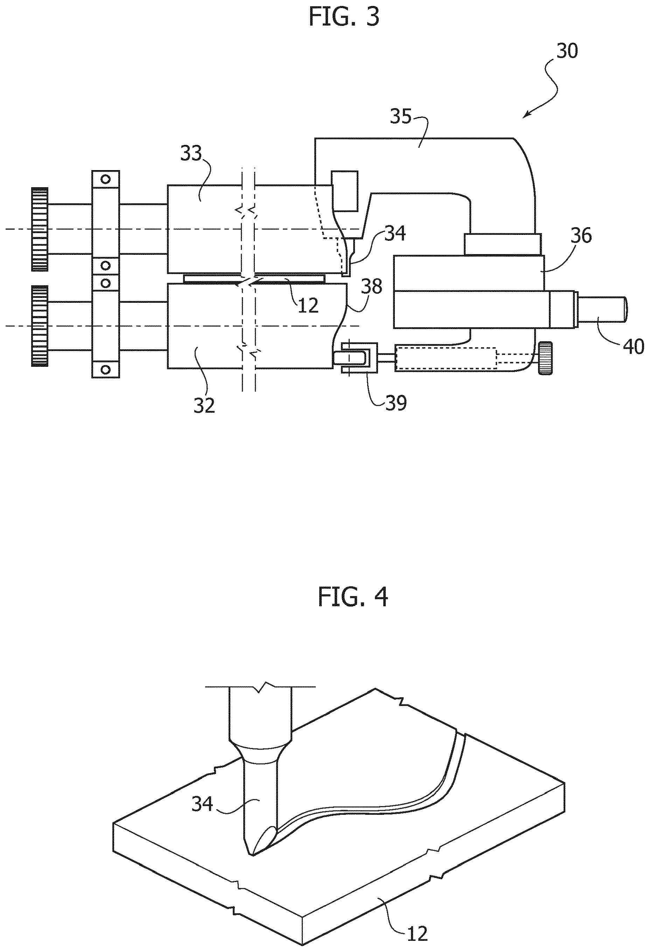

According to the peculiar feature of the invention each cutting tool consists of either an ultrasonic cutter or a laser beam cutter, both generally well known per se in the art of cutting, and only the first one of which is shown in the drawings and is referenced as 34. As shown in more detail in Figure 4 the ultrasonic cutter comprises a vertical sharpened sonodrode, in a fixed or rotatable configuration and for instance operated by a relatively low power at high frequency, which is displaced vertically and transversally to the back layer 12 for instance by a slide 35 in turn displaced over a guiding support 36 by means of an electric rotary motor 37 controlled electronically and of a conventional screw-and-nut transmission as shown in Figures 1 and 2. The slide 35 as well as energization of the sonotrode 34 are operated by a programmable electronic control unit in synchronism with the continuous (or intermittent) advancement of the layer, for instance by means of an encoder associated to one of the rollers 32, 33.

In the alternative embodiment shown in Figure 3 displacement of the slide 35 carrying the sonotrode 34 is carried out by a cam system including a cam profile 38 for instance provided at least on the end face of the lower roller 32 facing the cutting unit 30, and an adjustable cam follower 39 operatively connected to the slide 35. The cam follower 39 is urged against the cam profile 38 for instance by a presser device comprising a pneumatic spring 40.

The arrangement of the cutting system of the invention equipped with a laser cutter can be substantially same as the one disclosed in the above in connection with the ultrasonic cutter: in such a case a source is in a fixed position on the support 36 or 31 and the laser beam with a fixed optics or specific scanner if needed is displaced by the motor-driven slide 35 transversally to the travel direction of the substrate to be subjected to shaped cut of its longitudinal edges, or the slide 35 can be operated by the cam system 38-39. As a further alternative the laser source can be kept stationary and an optical system can be provided to focus the laser beam onto the continuous tape formed by the back layer 12 carrying the absorbent cores 28, along the desired curved trajectory defining the shaped cut.

The embodiment of the invention having the ultrasonic cutter is generally less expensive than the laser cutter: however the latter is more precise while affording higher cutting speed, though being more expensive.

Of course, without prejudice to the principle of the invention, the details of construction and the embodiments can be widely varied with respect to those described and illustrated, without thereby departing from the scope of the invention as defined by the claims that follow. Namely, the washable absorbent garments disclosed in connection with the cutting system and method of the invention is to be considered as only one example of sanitary articles and the like to which the invention can be applied.

## Claims

1. A system for the shaped cutting of sanitary articles, in particular of washable absorbent garments having a back layer (12) of washable fabric and the like, wherein said back layer (12) is fed longitudinally along a travel direction to a cutting unit (30) including at least one cutting tool (34) movable transversally to said travel direction so as to cut at least one longitudinal side edge of the back layer (12), in combination with the longitudinal displacement thereof, along a curved profile, wherein said cutting tool (34) is selected from the group of ultrasonic cutters and laser beam cutters.

2. The system of claim 1, wherein said back layer (12) is designed to form a pant structure having rear and front waist sections (14, 16) and a crotch section (18) intermediate between the rear and front waist sections (14, 16), wherein the cutting unit (30) comprises a pair of cutting tools (34) operating at both longitudinal side edges of the back layer (12) so as to provide said crotch sections (18).

3. The system of claim 1, comprising a slide (35) carrying said cutting tool (34) and actuator means (37; 38, 39) for displacing said slide (35) transversally to said travel direction.

4. The system of claim 3, wherein that said actuator means include an electronically controlled motor (37).

5. The system of claim 3, wherein said actuator means include mechanical cam means (38, 39).

6. The system of claim 5, comprising at least one rotatable transverse flat roller (32) over which said back layer (12) is longitudinally displaced, wherein said cam means include a cam profile (38) provided at one end of said roller (32) and a cam follower (39) operatively connected to said slide (35) and urged against said cam profile (38).

7. The system of claim 6, wherein said cam follower (39) is adjustable.

8. The system of claim 6 or 7, comprising presser means (40) to urge said cam follower (39) against said cam profile (38).

9. The system of any of the preceding claims, wherein said ultrasonic cutter includes a sharpened sonotrode (34) in a fixed or rotatable configuration.

10. A method for the shaped cutting of sanitary articles, in particular of washable absorbent garments having a back layer (12) of washable fabric forming a pant structure having rear and front waist sections (14, 16) and a crotch section (18) intermediate between the rear and front waist sections (14, 16), wherein said back layer (12) is fed longitudinally along a travel direction to a cutting unit (30) including cutting tools (34) movable transversally to said travel direction so as to cut each longitudinal side edge of back layer (12), in combination with the longitudinal displacement thereof, along curved profiles defining said crotch sections (18), wherein said cutting is carried out either by an ultrasonic cutter (34) or by a laser beam cutter operating on a roller (32) or on a flat anvil support.

11. The method of claim 10, wherein each cutting tool (34) is carried on a slide (36) displaced transversally to said travel direction.

12. The method of claim 11, wherein the displacement of said slide (36) is actuated by an electronically controlled motor (37).

13. The method of claim 11, wherein the displacement of said slide (36) is provided by cam means (38, 39).

14. The method of claim 13, wherein said back layer (12) is longitudinally displaced at least over a transverse rotary roller (32) or a flat anvil support, and wherein said cam means include a cam profile (38) provided at least at one end of said roller (32) and a cam follower (39) operatively connected to said slide (36) and urged against said cam profile (38).

15. Use of the system according to claim 1 for producing absorbent garments (10) the back layer (12) of which is forming a pant structure having rear and front waist sections (14, 16) and a crotch section (18) intermediate between the rear and front waist sections (14, 16).
